# EUROPEAN PATENT SPECIFICATION

(11) **EP 4 009 933 B1**
(45) Date of publication and mention of the grant of the patent: **22.05.2024**
(21) Application number: 20751151.0
(22) Date of filing: 06.08.2020
(51) Int. Cl.: A61H 7/00, A61H 23/02, A61M 21/02, A61M 21/00

(54) **APPARATUS AND METHOD FOR TACTILE STIMULATION OF HUMAN SKIN**
VORRICHTUNG UND VERFAHREN ZUR TAKTILEN STIMULATION DER MENSCHLICHEN HAUT
APPAREIL ET PROCÉDÉ DE STIMULATION TACTILE DE LA PEAU HUMAINE

(30) Priority: 07.08.2019 EP 19190486
(43) Date of publication of application: 15.06.2022
(73) Proprietor: Di Lernia, Daniele, 20017 Rho (MI) (IT)
(72) Inventor: Di Lernia, Daniele, 20017 Rho (MI) (IT)
(74) Representative: De Bortoli, Tiziano
(86) International application number: PCT/EP2020/072163
(87) International publication number: WO 2021/023827

(56) References cited:
- EP-A1- 2 879 643
- US-A1- 2012 259 255
- US-A1- 2013 041 296
- US-A1- 2014 228 721

## Description

### Technical field of the invention

The present invention relates, in general, to the technical field of tactile stimulation. In particular, the present invention relates to an apparatus and method for providing non-invasive interoceptive tactile stimulation of human skin for treating pathological conditions, such as, for example, pain both acute and chronic, anxiety, chronic stress, addictions, depression, post-traumatic stress disorder (PTSD) and emotional dysregulations in other pathologies such as autism, attention deficit hyperactivity disorder (ADHD) etc. Moreover, the inventive apparatus can also be used to improve well-being in healthy individuals, namely by enhancing their autonomic parasympathetic system and emotion regulation, and to promote more immersive experiences in simulated and/or virtual reality environments for both consumer (e.g. games) and clinical uses.

### Background of the invention

In highly social species, across the lifespan, touch plays a central role in the formation and maintenance of relationships. Between an infant and caregiver, within primate hierarchies and in romantic relationships, tactile interactions are rewarding, buffer physiological and psychological responses to stress and ultimately enhance well-being. For example, parental touch is a key regulator of an infant's physiological and behavioural arousal and, in adults, physical contact has been shown to modulate physiological responses to an acute stressor, to increase the autonomic wellbeing, and to reduce the perception of pain.

Several types of tactile perceptions exist, and they are processed by different areas of our brain, providing different psycho-physiological effects upon our body. Nonetheless, despite this centrality of touch to our bodily perception and well-being, to date, little attention has been paid to the neurophysiological basis of its beneficial effects. Among different types of touch, the interoceptive touch is a very specific secondary type of tactile perception that is processed by the interoceptive system and demonstrated a broad range of psychophysiological beneficial effects.

The study of the interoceptive system represents an emerging and promising topic in neuroscience, due to the meaning that interoceptive information has to the functioning of the organism. Interoceptive perceptions can be defined as the sense of the physiological status of the entire organism and they encompass a broad range of relevant biological functions that serve conscious and unconscious processes.

The central component of the interoceptive system is the anterior insular cortex (AIC) that receives information through a vast network of small unmyelinated fibers connected to the Lamina I spinothalamocortical pathway. These unmyelinated fibers compose a poly-modal afferent system that innervates the entire organism and report a wide range of inputs such as: hunger, thirst, pain, itch, temperature, muscle contraction, hormonal and immune activity, cardiorespiratory function, and a specific kind of touch, with unique neurophysiological effects.

The inputs are processed in the interoceptive matrix according to an explicit lateralization of the cerebral cortex. Specifically, the left and right insula of the cerebral cortex are usually coactive in the interoceptive system; nonetheless parasympathetic inputs are preferentially processed by the left insula, while sympathetic inputs are usually processed by the right insula.

Recent evidence identified interoceptive alterations in a broad range of clinical conditions, such as chronic pain, eating disorders, anxiety, depression, addictions, post traumatic stress disorders, insomnia, and several other conditions. This evidence suggests that a device able to stimulate of the interoceptive system could provide a therapeutic solution for such conditions.

As stated above, the interoceptive system is composed by a poly-modal afferent network of C-fibers. These fibers are unique and are able to collect a broad range of information. Interestingly, the C-fibers differentiate in free tactile arborizations on the superficial layer of the derma, creating a secondary touch system that is specifically connected to and processed by the interoceptive cortical areas.

C-Tactile afferents (CTs) are unmyelinated low threshold mechanoreceptors innervating the skin of mammals and they are uniquely found in not-glabrous skin.

In humans, their response characteristics have been elegantly mapped using single-unit microneurography recordings, establishing that C-Tactile afferents only respond to a precise low force and velocity skin temperature stimulus moving across their receptive field.

In particular, it has been found that C-Tactile afferents show the highest firing frequency to light stroking with a pressure force equal to or lower than 2.5 mN and within a stroking velocity between 1 and 10 cm/s, with an optimal velocity of 3 cm/s. Moreover, C-Tactile afferents exhibit a tendency to fatigue and a specific after-discharge pattern with a delayed acceleration effect.

Intriguingly, several clinical conditions demonstrated alterations in perceptions that were connected to C-Tactile afferents, suggesting an implication for interoceptive touch also in psychopathological functioning. As a further matter, human and animal models indicated that interoceptive touch can reduce and modulate pain perception, anxiety, and chronic stress, modulate µ-opioids and oxytocin response, enhance well-being and emotion regulation in healthy individuals, as well as promote embodiment in virtual and simulated environments.

The above considerations suggest a promising role of interoceptive tactile stimulation in different fields, from clinical applications to sensing technology and assessment; nevertheless, the same considerations underline the need for apparatuses that can supply interoceptive tactile stimulation, both for applied research and clinical treatments.

Such apparatuses actually exist configured to supply interoceptive tactile stimulation. However, the known apparatuses present some drawbacks.

Firstly, they are unable to automatically regulate the tactile stimulation based on the physical characteristics of the user's skin, and thus the stimulation treatment must be carried out by specialized personnel, in order to stimulate the C-Tactile afferents with the above indicated optimal stroking force and velocity values. Interoceptive tactile afferents respond exclusively to a narrow set of parameters that must be constantly monitored and tuned to ensure a proper activation of the interoceptive system and its following beneficial effects on the organism.

The physical characteristics of the user's skin, such as for example the thickness of the fat layer, the dimension of the body section to stimulate, the specific body morphological structure can interfere with the stimulation parameters, reducing or even cancelling the effects of the interoceptive tactile stimulation, which must be constantly maintained within strict parameters to express its therapeutic effects.

Second, the known apparatuses for interoceptive tactile stimulation are typically connected to a fixed setup, limiting the portability of the apparatuses in different locations, such as hospitals, laboratories and the like, and the possibility to apply stimulation to different body parts of a user.

A known stimulation apparatus is described, for example, in Di Lernia, D., P. Cipresso, E. Pedroli and G. Riva (2018). "Toward an Embodied Medicine: A Portable Device with Programmable Interoceptive Stimulation for Heart Rate Variability Enhancement. Sensors (Basel) 18(8).

Furthermore the document US 2013/041296 A1 discloses an apparatus for tactile stimulation of C-Tactile afferents comprising a casing configured to be positioned onto a user's skin and at least one tactile stimulation element carried by the casing and operated by a motor at a controlled velocity for generating at least one tactile stimulation.

The main object of the present invention is therefore to provide an apparatus and a method for tactile stimulation of C-Tactile afferents configured to overcome or at least reduce the drawbacks above mentioned with reference to the known tactile stimulation apparatuses.

More specifically, the main object of the present invention is to provide an apparatus for tactile stimulation of C-Tactile afferents configured to automatically regulate the tactile stimulation based on the physical characteristics of the user's skin, so as to stimulate the C-Tactile afferents with the above indicated optimal stroking force and velocity.

Another object of the present invention is to provide an apparatus for tactile stimulation of C-Tactile afferents configured to be portable in different locations, such as hospitals, laboratories and the like, and also configured to allow home treatment.

Another object of the present invention is to provide an apparatus for tactile stimulation of C-Tactile afferents configured to allow a plurality of apparatuses to be used for simultaneously stimulating different body parts of a user.

A further object of the present invention is to provide an apparatus for tactile stimulation of C-Tactile afferents which can be produced at competitive costs. The above-mentioned objects, and other objects that will better appear in the following of the present description, are achieved by an apparatus and a method for tactile stimulation of C-Tactile afferents as defined in the attached independent claims 1 and 15. Preferred characteristics of the apparatus and method for tactile stimulation of C-Tactile afferents are set forth in the dependent claims 2-14 and 16-20, respectively.

In a first aspect thereof, the present invention relates to an apparatus for tactile stimulation of C-Tactile afferents comprising a casing configured to be positioned onto a user's skin and at least one tactile stimulation element carried by the casing and operated by a motor at a controlled velocity for generating at least one tactile stimulation.

The apparatus is characterized in that it further comprises an electronic control unit and at least one pressure sensor in electrical communication with the electronic control unit to control the pressure force exerted by the at least one tactile stimulation element onto the user's skin by adjusting the position of the tactile stimulation element with respect to the user's skin based on a feedback signal transmitted from said at least one pressure sensor.

Preferably, the at least one tactile stimulation element is controlled to exert onto the user's skin a pressure force matching a value requested by the C-Tactile afferents.

In a second aspect thereof, the present invention relates to a method of generating at least one tactile stimulation comprising the steps of:
- operating at least one tactile stimulation element at a controlled velocity to generate at least one tactile stimulation;
- adjusting the position of the at least one tactile stimulation element with respect to the user's skin to control the pressure force exerted by the at least one tactile stimulation element onto the user's skin, based on a feedback signal transmitted by at least one pressure sensor.

Preferably, the at least one tactile stimulation element is lowered/raised to exert onto the user's skin a pressure force matching a value requested by the C-Tactile afferents.

Due to the above combination of features, in particular due to the presence of the one or more pressure sensors, the apparatus and the method defined above are able to automatically maintain precise stimulation parameters of the C-Tactile afferents, in particular the stroking force, regardless of the physical characteristics of the user's body.

Therefore, the apparatus according to the present invention advantageously allows the C-Tactile afferents to be stimulated with the optimal stroking force and velocity values, without the intervention of and the specialized personnel.

Advantageously, the apparatus for tactile stimulation of C-Tactile afferents of the present invention is so sized to be portable in different locations, for example at the user's home for home treatment, and to allow a plurality of apparatuses to be simultaneously used to stimulate different body parts of a user.

In a further aspect thereof, the present invention relates to the use of the apparatus and of the method defined above for producing pain analgesia and stress relief, but also for enhancing well-being and emotion regulation in healthy individuals and promoting embodiment in virtual and simulated environments. Hereafter, in the present description, and in the claims, the expression "tactile stimulation" is referred to stimulation of C-Tactile afferents or interoceptive tactile stimulation.

### Brief description of the drawings

Further features and advantages of the present invention will become more readily apparent from the following detailed description of a preferred embodiment of an apparatus and a method for generating at least one tactile stimulation, provided below, by way of non-limiting indication, with reference to the accompanying drawings. In the drawings:
- Figure 1 is a schematic top view of an apparatus for tactile stimulation according to a first embodiment of the present invention;
- Figure 2 is a schematic perspective view of the apparatus of Figure 1;
- Figure 3 is a schematic cross-sectional view of the apparatus of Figure 1, with the tactile stimulation element in the retracted or rest condition thereof;
- Figure 4 is a schematic cross-sectional view of the apparatus of Figure 1, with the tactile stimulation element in the extracted or operative condition thereof;
- Figure 5 schematically shows the way of interaction between the different components of the apparatus according to the present invention;
- Figure 6 is a schematic cross-sectional view of a second embodiment of the apparatus for tactile stimulation according to the present invention, with the tactile stimulation element in the retracted or rest condition thereof;
- Figure 7 is a schematic cross-sectional view of the apparatus of Figure 6, with the tactile stimulation element in the extracted or operative condition thereof;
- Figure 8 is an enlarged view of the particular circled with C in Figure 7; and
- Figure 9 is a flow chart, schematically showing the different steps of a method of tactile stimulation of C-Tactile afferents carried out with the apparatuses of Figures 1-8.

### Description of preferred embodiments of the invention

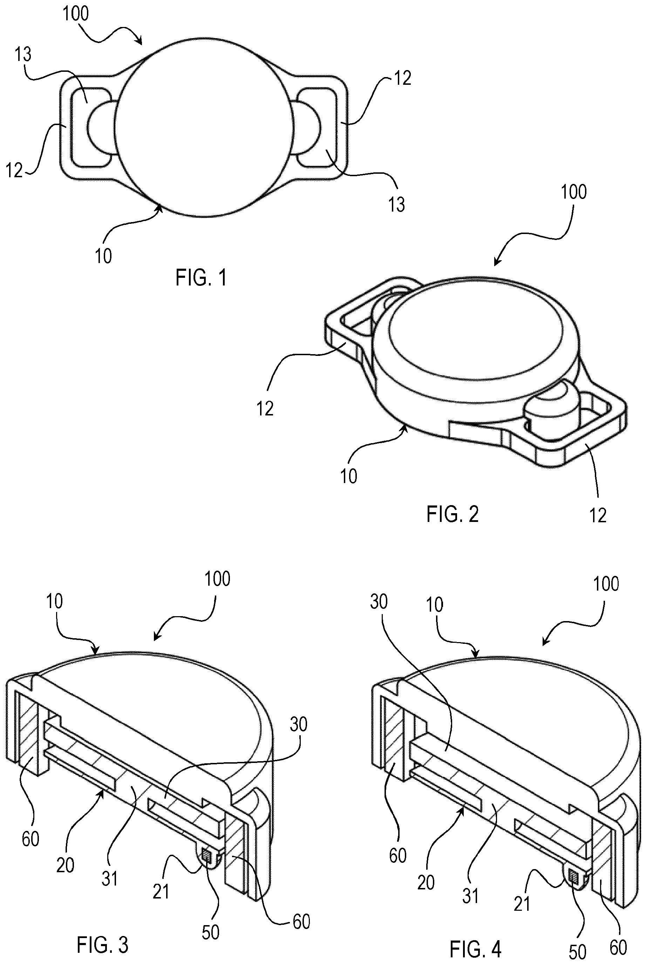

With reference to Figures 1 to 5, a tactile stimulation apparatus according to a first embodiment of the present invention is indicated, in general, with the reference numeral 100.

The apparatus 100 comprises a casing 10, which is configured to house the mechanical and electronical components of the apparatus, which will be described in detail below.

The casing 10 is preferably made of plastic material and is suitably dimensioned to be portable and easily positioned on the user's body part to be stimulated. For this purpose, the casing 10 comprises a pair of tabs 12, which laterally extend from the casing 10, opposite each other. Each tab 12 is provided with an aperture 13 through which a preferably elastic band (not shown) can pass to suitably position the apparatus onto the user's skin.

The apparatus 100 further comprises a tactile stimulation element 21 configured to stimulate C-Tactile afferents of the user's skin with predetermined stimulation parameters.

In the embodiment shown in Figures 1 to 4, the tactile stimulation element 21 projects downwards from a peripheral portion of a stimulation disk 20 supported by the casing 10, to contact, in use, the user's skin. Naturally, it is possible to provide a plurality of tactile stimulation elements 21, each downwardly projecting from the peripheral portion of the stimulation disk 20.

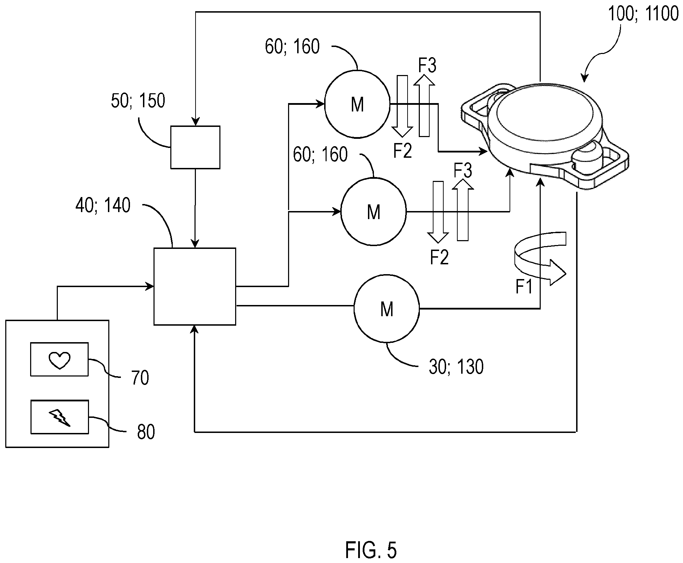

The stimulation disk 20 has preferably a circumference of 10 cm and is connected to a main shaft 31 of a motor 30, which drives the stimulation disk 20, and the tactile stimulation element 21 downwardly projecting therefrom, to generate at least one interoceptive tactile stimulation. Preferably, the motor 30 is a rotary motor, for example a stepper/servo rotary motor, preferably a piezoelectric rotary motor, which moves, preferably rotates (see arrow F1 in Figure 5) the tactile stimulation element 21 at a controlled velocity for generating a circular stimulation pattern.

In a preferred embodiment thereof, the tactile stimulation element 21 carried by the stimulation disk 20 has an oval-shape. This preferred configuration of the tactile stimulation element 21 allows a circular stimulation pattern at a specific calibrated velocity thus helps avoiding C-Tactile afferents fatigue and adaptation. C-Tactile afferents tend to exhibit fatigue and adaptation after 5s of continuous stimulation. The circular stimulation patter provided by the tactile stimulation element 21 mounted on the stimulation disk 20, allows to constantly change the site of stimulation, ensuring that CT afferent patches are directly stimulated only for short period of time (< 5s) avoiding fatigue and habituation of the receptors. As shown in greater detail in Figure 5, the apparatus 100 further comprises an electronic control unit 40, which is in electrical communication with the motor 30 to control the velocity of the tactile stimulation element 21, and thus the C-Tactile afferents stroking velocity. More specifically, and preferably, the motor 30 connected to the tactile stimulation element 21 is controlled by the electronic control unit 40 to produce an interoceptive tactile stimulation at a velocity between 1 cm/s to 10 cm/s, with optimal velocity set to 3 cm/s, velocities at which, how disclosed above, the CTs show the highest firing frequency.

The apparatus 100 further comprises at least one pressure sensor 50 configured to detect the pressure force exerted by the tactile stimulation element 21 onto the user's skin, during the operation of the apparatus.

As schematically illustrated in Figures 3 and 4, the apparatus 100 comprises a pressure sensor 50, which is embedded in the tactile stimulation element 21. Preferably, the pressure sensor 50 is in the form of micro load cell, pressure sensitive graphene polymers, or any other means able to detect the forces required by the interoceptive tactile stimulation.

As shown in greater detail in Figure 5, the pressure sensor 50 is in electrical communication with the electronic control unit 40, which in turn, utilizes a feedback signal transmitted by the pressure sensor 50 to adjust the position of the tactile stimulation element 21 with respect to the user's skin. More specifically, the electronic control unit 40 operates a further motor 60, preferably a pair of motors 60, to adjusting the position of the tactile stimulation element 21 with respect to the user's skin. More specifically, the tactile stimulation element 21 is lowered (see arrow F2) or raised (see arrow F3) by the further motor(s) to produce a pressure force lower than 40 mN, preferably equal to about 2.5 mN. In fact, as disclosed above, C-Tactile afferents show the highest firing frequency to light stroking with a pressure force equal to about 2.5 mN.

With reference to Figure 5, the electronic control unit 40 of the apparatus 100 can be electrically connected with one or more biosensors 70, 80, for example - but not limited to - ECG electric sensors, galvanic skin response sensors, temperature sensors, plethysmographic sensors, respiratory band sensors, graphene haptic sensors, which are configured to detect a physiological data of the user, for example hear rate, heart rate variability, sympathetic and parasympathetic responses, stress level, oxygen saturation, and to transmit the detected data to the electronic control unit 40. The electronic control unit 40, in turn, uses the received physiological data to regulate the tactile stimulation according to the user's physiological response. These biosensors can be either embedded in the apparatus 100, or the apparatus can interface with external biosensors such as smart fitness bracelets, fitness bands and other devices, to collect physiological data and modulate the stimulation accordingly.

The apparatus 100 further comprises a battery (not shown), preferably of the rechargeable type, for supplying power to the motors 30 and 60, the pressure sensor 50, the biosensors when embedded in the apparatus, and the electronic control unit 40. The battery is also housed in the casing 10.

The apparatus 100 can be equipped with a BLE (Bluetooth low energy) module connected to the electronic control unit 40. The BLE module allows wireless pairing with a smartphone, a personal computer, or any other electronic device that will host the user interface program.

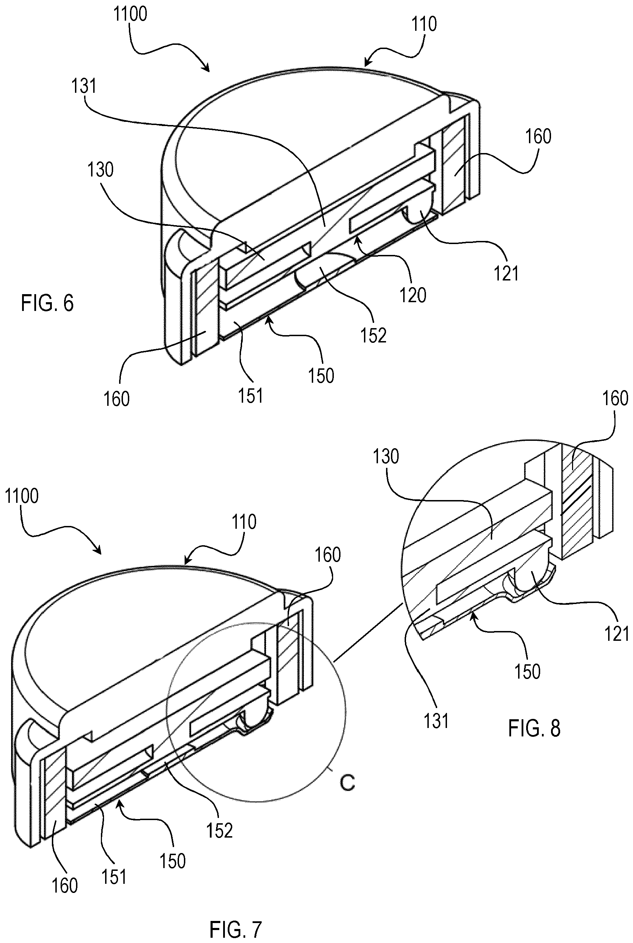

Figures 6 to 8 illustrate an apparatus for tactile stimulation according to a second embodiment of the present invention.

The apparatus, general designed with the reference numeral 1100, is similar to the apparatus 100 described above and illustrated in Figures 1-5, from which differs for the different configuration of the pressure sensor.

Therefore, the apparatus 1100 comprises a casing 110 and a stimulation disk 120 supported by the casing 110 and provided with a tactile stimulation element 121 downwardly projecting from a peripheral portion of the stimulation disk 120 to contact, in use, the user's skin. A plurality of tactile stimulation elements 121 can be also provided, each projecting from the peripheral portion of the stimulation disk 120.

The stimulation disk 120, and the at least one tactile stimulation element 121 downwardly projecting therefrom, are operated, preferably rotated by a motor 130 for generating a circular stimulation pattern. The tactile stimulation element 121 is the same of the stimulation element 21 and therefore it is not further described.

The apparatus 1100 comprises an electronic control unit 140 (see Figure 5) in electrical communication with the motor 130 to control the velocity of the tactile stimulation element 121 and thus the C-Tactile afferents stroking velocity.

The apparatus 1100 further comprises a pressure sensor 150, which unlike the pressure sensor 50 disclosed above is not embedded in the stimulation element, but is placed under the tactile stimulation element 121, so as to be interposed, in use, between the stimulation element 121 and the user's skin.

More specifically, the pressure sensor comprises a membrane 151 inside which a disk 152 made of graphene-composed polymer is housed. The membrane 151 is fixed to the casing 110 at its peripheral edge.

The membrane 151 is made of a flexible material and has a thickness lower than 1 mm. The disk 152 comprises electrical connections (not shown) to the electronic control unit 140, which, based on a feedback signal transmitted by the pressure sensor 150, operates a further motor 160, preferably a pair of motors 160, to adjust the position of the tactile stimulation element 121 with respect to the user's skin. More specifically, the tactile stimulation element 121 is lowered or raised by the further motor(s) 160 to produce on the C-Tactile afferents a stroking force lower than 40 mN, preferably equal to 2.5 mN.

The pressure sensor 150 made in the form of a graphene membrane advantageously allows to detect very small pressure values, typically used during the interoceptive tactile stimulation.

The apparatuses 100, 1100 according the first and second embodiments described above, further comprises a user interface (not shown), through which a user may select a set of stimulation parameters, more specifically stroking time, inter stimulus intervals, or pattern (frequency) of the stimulation. These stimulation parameters may be combined together thereby creating different types of interoceptive tactile stimulation.

In continuous stimulation, the apparatus 100, 1100, specifically the tactile stimulation element 21, 121, is controlled to provide a continuous interoceptive stimulus. The stroking time or duration of the stimulation may be stored in a memory, for example a flash memory, of the electronic control unit 40, 140; otherwise, the movement of the tactile stimulation element 21, 121 can be stopped at the appropriate time via a coded command selected through user interface.

In variance stimulation, the tactile stimulation element 21, 121 is controlled to provide a series of stimuli in a sequence. The duration of the single stimulus, the duration of the pause between stimuli, or both may be customized to address different therapeutic purposes.

The tactile stimulation element 21, 121 can also be continuously lowered and raised to produce on the user's skin a vibrational stimulation with a force greater than 40mN. The frequency of this continuous oscillatory movement is controlled by the motor(s) 60, 160 driven by the electronic control unit 40, 140 so as to allow a wide range of vibrational frequencies. In such a way, the apparatus 100, 1100 is also configured to produce proprioceptive tactile stimulation, in addition to the interoceptive tactile stimulation. Advantageously, proprioceptive tactile stimulation can also be used in alternate way with interoceptive tactile stimulation to enhance the effect of the latter, further avoiding habituation. The same goal could also be obtained by incorporating small vibro electrical motors in the apparatus 100, 1100 and connecting these vibro electric motors to the same control unit 40, 140 that coordinate/manage the interoceptive stimulation.

According to an alternative embodiment, the motor 30, 130 is a brushless PCB (printed circuit board) magnetic motor, comprising a plurality of conductive traces (not shown) embedded in different layers of the PCB. A series of electromagnets are thus formed, which are placed in a peripheral portion of the motor 30, 130, each electromagnet being individually controlled by the electronic control unit 40, 140. Permanent magnets (not shown) are provided in the stimulation disk 20, 120, which acts as a magnetic spool inside which the tactile stimulation element 21, 121 is mounted, and further comprises rotating electrical contacts (not shown).

During use of the apparatus 100, 1100, the magnetic spool/stimulation disk is trapped in a magnetic field and allowed to rotate on the peripheral portion of the casing 10, 110. The electronic control unit 40, 140 determines the alternation in the magnetic field within the electromagnets embedded in the motor 30, 130 and drives the magnetic spool/stimulation disk with the tactile stimulation element 21, 121 to rotate at the predetermined speed. Variations in the intensity and polarity of the magnetic field allow the apparatus 100, 1100 to lower and raise the magnetic spool/stimulation disk and the tactile stimulation element 21, 121, thereby regulating the pressure of the tactile stimulation element 21, 121 on the user's skin so as to match the predetermined force. The lowering and raising movement of the magnetic spool-tactile stimulation element assembly can be either counterbalanced by a spring or be entirely trapped in a magnetic field. This embodiment has the advantage to avoid mechanical gears, thereby preventing mechanical failures, and to facilitate the apparatus assembly due to the fact that such magnetic motors can be created directly within a printed circuit board (PCB motors), with current common industrial technology. Therefore, in this embodiment, the apparatus 100, 1100 results in less mechanical pieces, a smaller form factor, with the magnetic motor and the electronic components originating from the same printed circuit board. Moreover, the electromagnetic traces of the PCB motor can be configured in additional rings within the larger one, thereby allowing more than one stimulation disk and tactile stimulation element to be controlled at the same time, effectively multiplying the amount of interoceptive stimulation applied to the skin.

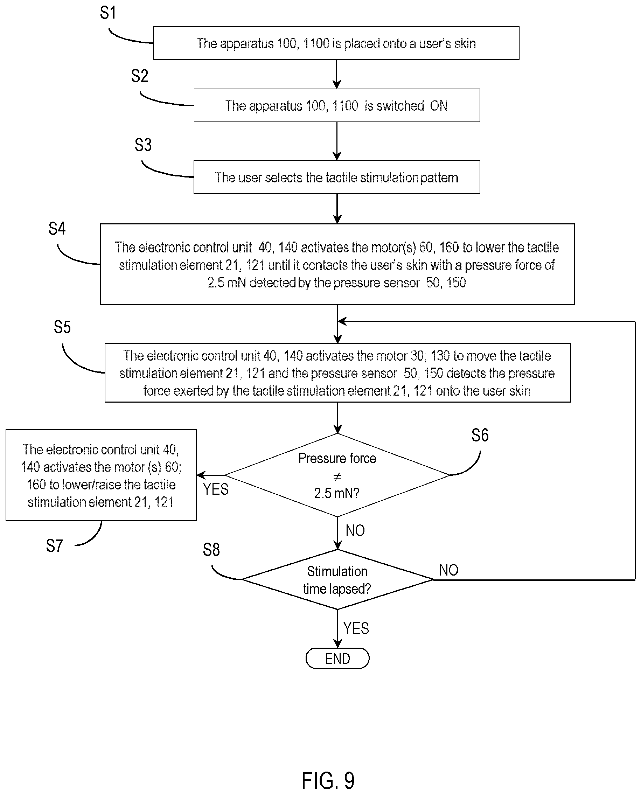

With reference to Figure 9, a method for tactile stimulation of C-Tactile afferents according to the present invention will now be described, the method being carried out by using the apparatus 100, 1100 described above and illustrated in Figures 1-8.

The method starts at step S1, in which the apparatus 100, 1100 is placed onto the user's skin, for example onto the area of the volar forearm, so that the tactile stimulation element 21, 121 is in contact with the user's skin.

The step S1 is followed by a step S2, in which the apparatus 100, 1100 is switched ON by the user, and by a step S3, in which the user selects the tactile stimulation pattern.

At this point, the apparatus 100, 1100 starts to perform the tactile stimulation based on the selected tactile stimulation pattern.

In particular, at step S4, the electronic control unit 40, 140 activates the further motor(s) 60, 160 to adjust the position of the tactile stimulation element 21, 121 with respect to the user's skin 21, 121 until it contacts the user's skin and at the same time the pressure sensor 50, 150 detects the pressure force exerted by the tactile stimulation element 21, 121 onto the user's skin. In particular, the tactile stimulation element 21, 121 is lowered until it contacts the user's skin. When the pressure force exerted by the tactile stimulation element 21, 121 onto the user's skin reaches a value lower than 40 mN, preferably a value set at 2.5 mN, the method proceeds to step S5, in which the electronic control unit 40, 140 activates the motor 30, 130 to move, preferably rotate the tactile stimulation element 21, 121 at a controlled velocity, preferably at a velocity between 1 cm/s to 10 cm/s, with optimal velocity set to 3 cm/s, and at the same time the pressure sensor 50, 150 detects the pressure force exerted by the tactile stimulation element 21, 121 onto the user's skin. During movement, the tactile stimulation element 21, 121 stimulates each point of the user's skin substantially for less than 5 seconds and this advantageously avoids the stimulated C-Tactile afferents to exhibit a tendency to fatigue.

If the pressure force detected by the pressure sensor 50, 150 is different from 2.5 mN (step S6, YES), the electronic control unit 40, 140 activates (S7) the further motor(s) 60, 160 to adjust the position of the tactile stimulation element 21, 121 with respect to the user's skin, so that the pressure force exerted by the stimulation element 21, 121 onto the underlying user's skin becomes equal to about 2.5 mN. More specifically, and preferably, the tactile stimulation element 21, 121 is lowered/raised with respect to the user's skin. In particular, and as an example, if the apparatus is used for the tactile stimulation of the user's volar forearm, when the tactile stimulation element 21, 121 moves along the arm, due to the shape of arm itself, the skin in contact with the tactile stimulation element 21, 121 may become thinner, so as to move away from the tactile stimulation element 21, 121, or vice versa the skin may become thicker, so as to get closer to the tactile stimulation element 21, 121. In the first case, the pressure sensor 50, 150 detects that the pressure force exerted by the tactile stimulation element 21, 121 onto the underlying skin decreases with respect to the set value of about 2.5 mN and thus the tactile stimulation element 21, 121 is lowered, to restore the set pressure force to its optimal value. In the second case, the pressure sensor 50, 150 detects that the pressure force exerted by the tactile stimulation element 21, 121 onto the underlying skin increases with respect to the set value of about 2.5 mN, corresponding to the optimal pressure force value for CT stimulation, and thus the tactile stimulation element 21, 121 is raised, to restore the set pressure force to its optimal value.

The method proceeds for the desired stimulation time (step S8, NO) and terminates when the stimulation time is lapsed (step S8, YES).

Preferably, the method further comprises a step in which one or more biosensors connected to the user detect respective physiological data of the user and transmit the detected data to the electronic control unit 40, 140. The electronic control unit 40, 140, in turn, uses the received physiological data to regulate the parameters (e.g. time, frequency, and pattern of the stimulation) of tactile stimulation according to the user's physiological response.

Preferably, the method further comprises, between steps S2 and S3, a pressure force calibration procedure.

During this calibration step, the tactile stimulation element 21, 121 is lowered to contact the user's skin until the pressure sensor detects the correct pressure force value for CT stimulation and the electronic control unit 40, 140 stores the angular and vertical position of the tactile stimulation element 21, 121, along with the pressure data. The tactile stimulation element 21, 121 is then rotated of 15° or more degrees and the calibration procedure is repeated till reaching 360° degrees of rotation.

Subsequently, the electronic control unit 40, 140 utilizes the angular and vertical position and the pressure data stored therein, to calculate a morphological map of the user's skin to evaluate and compensate specific surface distortions (e.g., muscles, fat layers, body different morphological conformations).

From the above description, the features of the apparatus and of the method for tactile stimulation of the present invention, as well as the advantages thereof, are evident.

In particular, the inventive apparatus and the method allow to automatically regulate the tactile stimulation based on the physical characteristics of the user's skin, so as to stimulate the C-Tactile afferents with the above indicated optimal pressure force and velocity.

Further, due to its small size, the inventive apparatus is portable in different locations, such as hospitals, laboratories and the like, and may be also used in home treatments.

Moreover, due to its small size, a plurality of apparatuses may be used for simultaneously stimulating different body parts of a user. In this case all the apparatuses are connected to a personal computer or a remote server, preferably via wireless connections. In this configuration, the personal computer or the remote server can independently communicate with each apparatus to coordinate the stimulation upon large parts of the body. This method will allow to amplify the effect of the tactile stimulation, activating a large quantity of C-Tactile afferents at the same time, enhancing the therapeutic effect.

The person skilled in the art, in order to satisfy specific requirements, may make modifications to the embodiments of the apparatus for interoceptive tactile stimulation described above and/or replace parts described with equivalent parts, as long as they fall within the scope of the invention as defined in the attached claims.

For example, although separate motors are used for controlling the movement and the lowering/raising of the stimulation element, a single motor may be used to control the stimulation element.

## Claims

1. Apparatus (100; 1100) for tactile stimulation of C-Tactile afferents comprising a casing (10; 110) configured to be positioned onto a user's skin and at least one tactile stimulation element (21; 121) carried by the casing (100; 110) and operated by a motor (30; 130) at a controlled velocity for generating at least one tactile stimulation;
**characterized in that** the apparatus (100; 1100) further comprises an electronic control unit (40; 140) and at least one pressure sensor (50; 150) in electrical communication with the electronic control unit (40; 140) to control the pressure force exerted by said at least one tactile stimulation element (21; 121) onto the user's skin by adjusting the position of the tactile stimulation element (21; 121) with respect to the user's skin, based on a feedback signal transmitted by said at least one pressure sensor (50; 150).

2. Apparatus (100; 1100) according to claim 1, further comprising a stimulation disk (20; 120) supported by the casing (10; 110), said at least one tactile stimulation element (21; 121) consisting of a tactile stimulation element (21; 121) projecting downwards from a peripheral portion of the stimulation disk (20; 120).

3. Apparatus (100) according to claim 2, wherein said at least one pressure sensor (50) is embedded in said at least one stimulation element (21).

4. Apparatus (1100) according to claim 2, wherein said at least one pressure sensor (150) is provided under the tactile stimulation element (121) so as to be interposed, in use, between the at least one tactile stimulation element (121) and the user's skin.

5. Apparatus (1100) according to claim 4, wherein said at least one pressure sensor (150) comprises a membrane (151) inside with a disk (152) made of graphene-composed polymer is housed, with said membrane (151) being connected to the casing (110) at a peripheral edge thereof.

6. Apparatus (100; 1100) according to any one of the preceding claims, wherein said at least one tactile stimulation element (21; 121) is controlled for adjusting the position of the tactile stimulation element (21; 121) with respect to the user's skin by at least one further motor (60, 160) in electrical connection with the electronic control unit (40, 140) to lower/raise the tactile stimulation element (21; 121) with respect to the user's skin.

7. Apparatus (100; 1100) according to any one of the preceding claims, wherein the at least one tactile stimulation element (21, 121) is controlled by the electronic control unit (40; 140) to move at a velocity between about 1 cm/s to about 10 cm/s, preferably at a velocity of about 3 cm/s.

8. Apparatus (100; 1100) according to any one of the preceding claims, wherein the at least one tactile stimulation element (21, 121) is controlled by the electronic control unit (40; 140) to produce a pressure force onto the user's skin lower than 40mN, and preferably equal to about 2.5 mN.

9. Apparatus (100; 1100) according to any one of the preceding claims, wherein the electronic control unit (40; 140) can be electrically connected with one or more biosensors (70; 80), which are configured to detect a physiological data of the user and transmit the detected data to the electronic control unit (40; 140), which, in turn, uses the received physiological data to regulate the tactile stimulation according to the user's physiological response.

10. Apparatus (100; 1100) according to any one of the preceding claims, wherein the motor (30; 130) is a PCB magnetic motor, the apparatus further comprising a plurality of conductive traces embedded in different layers of the PCB thereby producing a series of electromagnets placed in a peripheral portion of the motor (30, 130), each electromagnet being individually controlled by the electronic control unit (40, 140).

11. Apparatus (100; 1100) according to claim 10, further comprising permanent magnets provided in the stimulation disk (20, 120), which thus acts as a magnetic spool inside which the tactile stimulation element (21, 121) is mounted, the magnetic spool-tactile stimulation element assembly be allowed to rotate by alternating the magnetic field within the electromagnets embedded in the PCB magnetic motor (30; 130), and the magnetic spool-tactile stimulation element assembly be allowed to lower and raise by varying the intensity and polarity of the magnetic field.

12. Apparatus (100; 1100) according to claim 10 or 11, further comprising a spring configured to counterbalance the lowering and raising movement of the magnetic spool-tactile stimulation element assembly.

13. Apparatus (100; 1100) according to any one of the preceding claims, further comprising one or more vibro electrical motors connected to the control unit (40, 140) to lower and raise the tactile stimulation element (21; 121) to produce on the user's skin a vibrational stimulation greater than 40mN.

14. Apparatus (100; 1100) according to any one of the preceding claims for use in generating at least one adjustable tactile stimulation for generating pain analgesia and stress relief, to improve well-being in healthy individuals or to promote more immersive experiences in simulated environments for both consumer and clinical uses.

15. Apparatus according to any one of the preceding claims, wherein the electronic control unit is configured to:
- lower the at least one tactile stimulation element (21; 121) to contact the user's skin until the at least one sensor pressure (50; 150) detects a pressure force value of about 2.5 mN;
- store an angular and vertical position of the at least one tactile stimulation element (21; 121), along with pressure data;
- rotate the at least one tactile stimulation element (21; 121) of 15 or more degrees till reaching 360 degrees of rotation; and
- use the stored angular and vertical position and the pressure data to calculate a morphological map of the user's skin to evaluate and compensate specific surface distortions.

## Patentansprüche

1. Einrichtung (100; 1100) zur taktilen Stimulation von C-taktilen-Afferenzen, umfassend ein Gehäuse (10; 110), das konfiguriert ist, um auf der Haut eines Benutzers positioniert zu werden, und zumindest ein taktiles Stimulationselement (21; 121), das von dem Gehäuse (100; 110) getragen, und von einem Motor (30; 130) mit einer gesteuerten Geschwindigkeit zum Erzeugen zumindest einer taktilen Stimulation betrieben wird;
**dadurch gekennzeichnet, dass** die Einrichtung (100; 1100) weiter eine elektronische Steuereinheit (40; 140) und zumindest einen Drucksensor (50; 150) in elektrischer Kommunikation mit der elektronischen Steuereinheit (40; 140) umfasst, um die Druckkraft, die durch das zumindest eine taktile Stimulationselement (21; 121) auf die Haut des Benutzers ausgeübt wird, durch Anpassen der Position des taktilen Stimulationselements (21; 121) in Bezug auf die Haut des Benutzers basierend auf einem von dem zumindest einen Drucksensor (50; 150) übertragenen Rückkopplungssignal zu steuern.

2. Einrichtung (100; 1100) nach Anspruch 1, weiter eine Stimulationsscheibe (20; 120) umfassend, welche von dem Gehäuse (10; 110) getragen wird, wobei das zumindest eine taktile Stimulationselement (21; 121) aus einem taktilen Stimulationselement (21; 121) besteht, welches von einem Umfangsabschnitt der Stimulationsscheibe (20; 120) nach unten hervorsteht.

3. Einrichtung (100) nach Anspruch 2, wobei der zumindest eine Drucksensor (50) in das zumindest eine Stimulationselement (21) eingebettet ist.

4. Einrichtung (1100) nach Anspruch 2, wobei der zumindest eine Drucksensor (150) unter dem taktilen Stimulationselement (121) bereitgestellt ist, um im Gebrauch zwischen dem zumindest einen taktilen Stimulationselement (121) und der Haut des Benutzers eingefügt zu sein.

5. Einrichtung (1100) nach Anspruch 4, wobei der zumindest eine Drucksensor (150) eine Membran (151) umfasst, in deren Inneren eine Scheibe (152), die aus einem Graphen-Polymer gefertigt ist, eingehaust ist, wobei die Membran (151) mit dem Gehäuse (110) an dessen Umfangsrand verbunden ist.

6. Einrichtung (100; 1100) nach einem der vorstehenden Ansprüche, wobei das zumindest eine taktile Stimulationselement (21; 121) zum Anpassen der Position des taktilen Stimulationselements (21; 121) in Bezug auf die Haut des Benutzers durch zumindest einen weiteren Motor (60, 160) gesteuert wird, welcher in elektrischer Verbindung mit der elektronischen Steuereinheit (40, 140) steht, um das taktile Stimulationselement (21; 121) in Bezug auf die Haut des Benutzers abzusenken/anzuheben.

7. Einrichtung (100; 1100) nach einem der vorstehenden Ansprüche, wobei das zumindest eine taktile Stimulationselement (21, 121) von der elektronischen Steuereinheit (40; 140) gesteuert wird, um sich mit einer Geschwindigkeit zwischen etwa 1 cm/s und 10 cm/s, vorzugsweise mit einer Geschwindigkeit von etwa 3 cm/s zu bewegen.

8. Einrichtung (100; 1100) nach einem der vorstehenden Ansprüche, wobei das zumindest eine taktile Stimulationselement (21, 121) durch die elektronische Steuereinheit (40; 140) gesteuert wird, um eine Druckkraft auf die Haut des Benutzers zu erzeugen, welche geringer als 40 mN, und vorzugsweise etwa gleich 2,5 mN ist.

9. Einrichtung (100; 1100) nach einem der vorstehenden Ansprüche, wobei die elektronische Steuereinheit (40; 140) mit einem oder mehreren Biosensoren (70; 80) elektrisch verbunden werden kann, die konfiguriert sind, um physiologische Daten des Benutzers zu erkennen und die erkannten Daten an die elektronische Steuereinheit (40; 140) zu übertragen, welche im Gegenzug die empfangenen physiologischen Daten verwendet, um die taktile Stimulation gemäß der physiologischen Reaktion des Benutzers zu regeln.

10. Einrichtung (100; 1100) nach einem der vorstehenden Ansprüche, wobei der Motor (30; 130) ein PCB-Magnetmotor ist und die Einrichtung weiter eine Vielzahl von Leiterbahnen umfasst, welche in verschiedene Schichten der PCB eingebettet sind, wodurch eine Reihe von Elektromagneten erzeugt wird, welche in einem Umfangsabschnitt des Motors (30, 130) platziert sind, wobei jeder Elektromagnet einzeln von der elektronischen Steuereinheit (40, 140) gesteuert wird.

11. Einrichtung (100; 1100) nach Anspruch 10, weiter Permanentmagnete umfassend, welche in der Stimulationsscheibe (20, 120) bereitgestellt sind, die somit als Magnetspule fungiert, in deren Inneren das taktile Stimulationselement (21, 121) montiert ist, wobei es der Baugruppe aus Magnetspule und taktilem Stimulationselement erlaubt ist, sich durch Abwechseln des Magnetfelds innerhalb der in dem PCB-Magnetmotor (30; 130) eingebetteten Elektromagneten zu drehen, und es der Baugruppe aus Magnetspule und taktilem Stimulationselement erlaubt ist, durch Variieren der Intensität und Polarität des Magnetfeldes abgesenkt und angehoben zu werden.

12. Einrichtung (100; 1100) nach Anspruch 10 oder 11, weiter eine Feder umfassend, die konfiguriert ist, um die Absenk- und Anhebebewegung der Baugruppe aus Magnetspule und taktilem Stimulationselement auszugleichen.

13. Einrichtung (100; 1100) nach einem der vorstehenden Ansprüche, weiter einen oder mehrere Vibrationselektromotoren umfassend, welche mit der Steuereinheit (40, 140) verbunden sind, um das taktile Stimulationselement (21; 121) abzusenken und anzuheben, um auf der Haut des Benutzers eine Vibrationsstimulation von mehr als 40 mN zu erzeugen.

14. Einrichtung (100; 1100) nach einem der vorstehenden Ansprüche zur Verwendung beim Erzeugen zumindest einer anpassbaren taktilen Stimulation zum Erzeugen von Schmerzanalgesie und Stressabbau, zur Verbesserung des Wohlbefindens gesunder Personen oder zur Förderung immersiverer Erfahrungen in simulierten Umgebungen sowohl für den Verbraucher- als auch den klinischen Gebrauch.

15. Einrichtung nach einem der vorstehenden Ansprüche, wobei die elektronische Steuereinheit konfiguriert ist, um:
- das zumindest eine taktile Stimulationselement (21; 121) abzusenken, um die Haut des Benutzers zu berühren, bis der zumindest eine Drucksensor (50; 150) einen Druckkraftwert von etwa 2,5 mN erkennt;
- eine Winkel- und Vertikalposition des zumindest einen taktilen Stimulationselements (21; 121) zusammen mit Druckdaten zu speichern;
- das zumindest eine taktile Stimulationselement (21; 121) um 15 Grad oder mehr zu drehen, bis eine Drehung von 360 Grad erreicht ist; und
- die gespeicherte Winkel- und Vertikalposition und die Druckdaten zu verwenden, um eine morphologische Karte der Haut des Benutzers zu berechnen, um spezifische Oberflächenverzerrungen zu bewerten und zu kompensieren.

## Revendications

1. Appareil (100 ; 1100) pour une stimulation tactile d'afférents C-tactiles comprenant un boîtier (10 ; 110) configuré pour être positionné sur la peau d'un utilisateur et au moins un élément de stimulation tactile (21 ; 121) porté par le boîtier (100 ; 110) et actionné par un moteur (30 ; 130) à une vitesse régulée pour générer au moins une stimulation tactile ; **caractérisé en ce que** l'appareil (100 ; 1100) comprend en outre une unité de commande électronique (40 ; 140) et au moins un capteur de pression (50 ; 150) en communication électrique avec l'unité de commande électronique (40 ; 140) pour commander la force de pression exercé par ledit au moins un élément de stimulation tactile (21 ; 121) sur la peau de l'utilisateur en ajustant la position de l'élément de stimulation tactile (21 ; 121) par rapport à la peau de l'utilisateur, sur la base d'un signal de rétroaction transmis par ledit au moins un capteur de pression (50 ; 150).

2. Appareil (100; 1100) selon la revendication 1, comprenant en outre un disque de stimulation (20 ; 120) supporté par le boîtier (10 ; 110), ledit au moins un élément de stimulation tactile (21 ; 121) étant composé d'un élément de stimulation tactile ( 21 ; 121) faisant saillie vers le bas depuis une partie périphérique du disque de stimulation (20 ; 120).

3. Appareil (100) selon la revendication 2, dans lequel ledit au moins un capteur de pression (50) est intégré dans ledit au moins un élément de stimulation (21).

4. Appareil (1100) selon la revendication 2, dans lequel ledit au moins un capteur de pression (150) est disposé sous l'élément de stimulation tactile (121) de manière à être intercalé, en utilisation, entre le au moins un élément de stimulation tactile (121) et la peau de l'utilisateur.

5. Appareil (1100) selon la revendication 4, dans lequel ledit au moins un capteur de pression (150) comprend une membrane (151) à l'intérieur avec un disque (152) réalisé en polymère composé de graphène, ladite membrane (151) étant reliée au boîtier (110) au niveau d'un bord périphérique de celui-ci.

6. Appareil (100 ; 1100) selon l'une quelconque des revendications précédentes, dans lequel ledit au moins un élément de stimulation tactile (21 ; 121) est commandé pour ajuster la position de l'élément de stimulation tactile (21 ; 121) par rapport à la peau de l'utilisateur par au moins un moteur supplémentaire (60, 160) en liaison électrique avec l'unité de commande électronique (40, 140) pour abaisser/lever l'élément de stimulation tactile (21 ; 121) par rapport à la peau de l'utilisateur.

7. Appareil (100 ; 1100) selon l'une quelconque des revendications précédentes, dans lequel le au moins un élément de stimulation tactile (21, 121) est commandé par l'unité de commande électronique (40 ; 140) pour se déplacer à une vitesse comprise entre environ 1 cm/s et environ 10 cm/s, de préférence à une vitesse d'environ 3 cm/s.

8. Appareil (100 ; 1100) selon l'une quelconque des revendications précédentes, dans lequel le au moins un élément de stimulation tactile (21, 121) est commandé par l'unité de commande électronique (40 ; 140) pour produire une force de pression sur la peau de l'utilisateur inférieure à 40 mN, et de préférence égale à environ 2,5 mN.

9. Appareil (100 ; 1100) selon l'une quelconque des revendications précédentes, dans lequel l'unité de commande électronique (40 ; 140) peut être raccordée électriquement à un ou plusieurs biocapteurs (70 ; 80), qui sont configurés pour détecter une donnée physiologique de l'utilisateur et transmettre la donnée détectée à l'unité de commande électronique (40 ; 140), qui, à son tour, utilise la donnée physiologique reçue pour réguler la stimulation tactile en fonction de la réponse physiologique de l'utilisateur.

10. Appareil (100 ; 1100) selon l'une quelconque des revendications précédentes, dans lequel le moteur (30 ; 130) est un moteur magnétique à carte PCB, l'appareil comprenant en outre une pluralité de traces conductrices intégrées dans différentes couches de la carte PCB, ce qui permet de produire une série d'électroaimants placés dans une partie périphérique du moteur (30, 130), chaque électro-aimant étant commandé individuellement par l'unité électronique de commande (40, 140)

11. Appareil (100; 1100) selon la revendication 10, comprenant en outre des aimants permanents disposés dans le disque de stimulation (20, 120), qui agit ainsi comme une bobine magnétique à l'intérieur de laquelle l'élément de stimulation tactile (21, 121) est monté, l'ensemble bobine magnétique-élément de stimulation tactile étant autorisé à tourner en alternant le champ magnétique à l'intérieur des électro-aimants intégrés dans le moteur magnétique à carte PCB (30 ; 130), et l'ensemble bobine magnétique-élément de stimulation tactile étant autorisé à s'abaisser et s'élever en faisant varier l'intensité et la polarité du champ magnétique.

12. Appareil (100 ; 1100) selon la revendication 10 ou 11, comprenant en outre un ressort configuré pour contrebalancer le mouvement d'abaissement et d'élévation de l'ensemble bobine magnétique-élément de stimulation tactile.

13. Appareil (100 ; 1100) selon l'une quelconque des revendications précédentes, comprenant en outre un ou plusieurs vibromoteurs électriques raccordés à l'unité de commande (40, 140) pour abaisser et lever l'élément de stimulation tactile (21 ; 121) pour produire sur la peau de l'utilisateur une stimulation vibratoire supérieure à 40 mN.

14. Appareil (100 ; 1100) selon l'une quelconque des revendications précédentes, destiné à être utilisé pour générer au moins une stimulation tactile ajustable pour générer une analgésie contre la douleur et un soulagement du stress, pour améliorer le bien-être d'individus en bonne santé ou pour promouvoir des expériences plus immersives dans des environnements simulés pour des utilisations grand public et cliniques.

15. Appareil selon l'une quelconque des revendications précédentes, dans lequel l'unité de commande électronique est configurée pour :
- abaisser le au moins un éléments de stimulation tactile (21 ; 121) pour qu'il vienne en contact avec la peau de l'utilisateur jusqu'à ce que le au moins un capteur de pression (50 ; 150) détecte une valeur de force de pression d'environ 2,5 mN ;
- stocker une position angulaire et verticale du au moins un élément de stimulation tactile (21 ; 121), conjointement avec des données de pression ;
- faire tourner le au moins un élément de stimulation tactile (21 ; 121) de 15 degrés, ou plus, jusqu'à atteindre 360 degrés de rotation ; et
- utiliser la position angulaire et verticale stockée et les données de pression pour calculer une carte morphologique de la peau de l'utilisateur pour évaluer et compenser des distorsions de surface spécifiques.
